# DEMANDE DE BREVET EUROPEEN

(11) **EP 0 955 034 A1**
(43) Date de publication de la demande: **10.11.1999**
(21) Numéro de dépôt: 99420086.3
(22) Date de dépôt: 02.04.1999
(51) Int. Cl.: A61K 7/155

(54) **Assemblage de matières thermoplastiques pour épilation**

(30) Priorité: 06.04.1998 FR 9804509
(71) Demandeur: SEB S.A., 69130 Ecully (FR)
(72) Inventeur: Cathaud, Muriel, 69780 Toussieu (FR); Paget, Monique, Optevoz, 38460 Cremieu (FR)

(57) **Abrégé**

- L'invention concerne un assemblage de matières thermoplastiques pour épilation.
- Conformément à l'invention il comporte un noyau (1) composé de cire à épiler et une couche d'enrobage (2) recouvrant au moins partiellement ledit noyau, ladite couche étant formée par une matière dont le point de ramollissement est plus élevé que celui de la cire dudit noyau.

## Description

La présente invention est relative au domaine technique des cires dites basses températures utilisées pour l'épilation. Cette catégorie de cires est utilisée dans des distributeurs de cire tels que par exemple des applicateurs de cire à rouleau.

Par matières thermoplastiques pour épilation, on entend toute matière qui se ramollit sous l'effet de l'élévation de la température. Cela comprend, d'une part, les cires cristallisées passant, sous l'influence de la température, de l'état solide cristallisé à l'état liquide et présentant une fusion nette, telles les cires végétales, par exemple à base de colophane. Ce même terme fait, d'autre part, également référence aux cires amorphes ou non cristallisées, présentant une fusion pâteuse, par exemple les cires au sucre ou à base d'un polymère non cristallin.

De telles cires sont déposées en couche mince sur la peau à l'aide d'un distributeur après avoir été fondues. La couche de cire une fois solidifiée emprisonne les poils et peut être retirée. Certaines cires, telles que celles connues du document EP 0 299 816, présentent l'avantage d'être autoporteuses. Avec de telles cires la couche de cire une fois solidifiée peut être retirée sans utiliser de support tel qu'une bande de cellophane.

Un distributeur de cire à épiler du type applicateur à rouleau est connu du document EP 0 629 366. Cet appareil comporte un réservoir associé à des moyens de chauffe et un rouleau prévu pour distribuer la cire fondue en couche mince sur la peau. La cire utilisée dans de tels distributeurs se présente généralement sous la forme de grains, par exemple des grains de forme sensiblement ellipsoïdale aplatie dont le diamètre est de l'ordre de 4 à 5 mm et la hauteur de 2 à 3 mm, de pastilles ou encore de pains.

Les cires dites basse température présentent, dans certaines conditions d'usage ou de stockage, des inconvénients liés à la fusion des couches de surface. Ces conditions sont par exemple rencontrées lors de la manipulation de la cire en vue du remplissage du réservoir d'un distributeur. La température des mains est suffisante pour entraîner le ramollissement de la surface des grains, des pastilles ou des pains de cire. Cette surface devient poisseuse et/ou graisseuse, ce qui rend la manipulation peu agréable. Ces conditions peuvent être également rencontrées lorsque les températures de stockage préconisées ne sont pas respectées. Les grains de cire peuvent alors s'agglomérer, ce qui rend leur utilisation ultérieure moins aisée. Plus généralement, le ramollissement de la cire entraîne, sous l'action de contraintes mécaniques extérieures, une modification de la géométrie des morceaux de cire ; cet inconvénient est particulièrement gênant lors de l'utilisation de pains de cire dans les applicateurs de cire comportant un dispositif chauffant à travers lequel le pain de cire passe en se ramollissant.

La présente invention vise à fournir des matières thermoplastiques pour épilation remédiant aux inconvénients précités.

Les objets assignés à l'invention sont atteints avec un assemblage de matières thermoplastiques pour épilation, caractérisé en ce qu'il comporte un noyau composé de cire à épiler et une couche d'enrobage recouvrant au moins partiellement ledit noyau, ladite couche étant formée par une matière dont le point de ramollissement est plus élevé que celui de la cire dudit noyau.

Ainsi les grains, pastilles ou pains de matière thermoplastique réalisés selon cet assemblage présentent une tenue en température améliorée. De plus cette disposition permet d'utiliser pour les matières du noyau des matières dont le point de ramollissement est moins élevé que celui des cires utilisées usuellement dans les applicateurs de cire à épiler. Il est ainsi envisageable d'utiliser des matières thermoplastiques présentant un point de ramollissement inférieur à 60°C pour le noyau.

Selon un premier mode de réalisation, la couche d'enrobage recouvre entièrement le noyau.

Cette disposition est particulièrement adaptée au conditionnement de la matière thermoplastique pour épilation sous forme de pastilles ou de grains. Les conditions de manipulation sont améliorées car les grains ou pastilles risquent moins de s'agglomérer suite à une exposition à une température plus élevée que les conditions ordinaires de stockage des matières thermoplastiques pour épilation dites basses température connues à ce jour. Il en résulte une simplification des conditions de stockage.

Selon un deuxième mode de réalisation, la couche d'enrobage est discontinue et laisse apparaître à la surface de l'assemblage de matières thermoplastiques le noyau.

Cette disposition est particulièrement adaptée au conditionnement de la matière thermoplastique pour épilation sous forme de pain.

Avantageusement le pain comporte deux faces d'extrémité et au moins une face latérale.

Cette disposition est bien adaptée à l'introduction du pain dans le réservoir d'un distributeur de cire. De manière courante le pain est de forme sensiblement parallélépipédique et comporte deux faces d'extrémité et quatre faces latérales.

Avantageusement la couche d'enrobage recouvre entièrement la ou les faces latérales.

Cette disposition permet d'une part la manipulation du pain sans présenter les désagréments liés au contact avec la cire à épiler, et d'autre part de former une enveloppe assurant une meilleure tenue mécanique du pain lorsque celui-ci rentre en contact avec une source chauffante, par exemple lors que le pain est utilisé dans un distributeur comportant un dispositif chauffant au travers duquel le pain passe à travers en se ramollissant. Le pain peut ainsi conserver ses caractéristiques dimensionnelles.

Avantageusement l'une au moins des faces d'extrémité laisse déboucher le noyau.

Cette disposition permet de commencer à fondre le noyau sans nécessiter au préalable de fondre la couche d'enrobage, en introduisant la face correspondante dans le distributeur en regard du dispositif chauffant.

L'invention sera mieux comprise à l'étude de modes de réalisation pris à titre nullement limitatifs et illustrés dans les figures annexées dans lesquelles :
- la figure 1 est une vue schématique en coupe longitudinale d'un premier mode de réalisation de l'assemblage de matières thermoplastiques pour épilation selon l'invention, sous une forme de pastille,
- la figure 2 est une vue schématique en perspective d'un deuxième mode de réalisation de l'assemblage de matières thermoplastiques pour épilation selon l'invention, sous une forme de pain.

L'assemblage de matières thermoplastiques pour épilation selon l'invention comporte un noyau 1 composé de cire à épiler, ainsi qu'une couche d'enrobage 2 recouvrant au moins partiellement ledit noyau, ladite couche étant formée par une matière dont le point de ramollissement est plus élevé que celui de la cire du noyau 1.

De préférence le point de ramollissement de la matière formant la couche d'enrobage 2 est supérieur d'au moins 5°C par rapport au point de ramollissement de la matière formant le noyau 1, et avantageusement d'au moins 10°C.

Des exemples de matières sont donnés ci-après pour la cire à épiler du noyau 1 et pour la couche d'enrobage 2.

L'épaisseur de la couche 2 est choisie de manière à protéger le noyau 1 des agressions extérieures telles qu'un apport de chaleur par exemple lors de la préhension dudit assemblage, sans pour autant affecter de façon trop importante les propriétés mécaniques des matières thermoplastiques utilisées dans le noyau 1. Avantageusement l'épaisseur de la couche d'enrobage 2 disposée sur le noyau 1 est sensiblement constante, et de préférence inférieure à 1 mm.

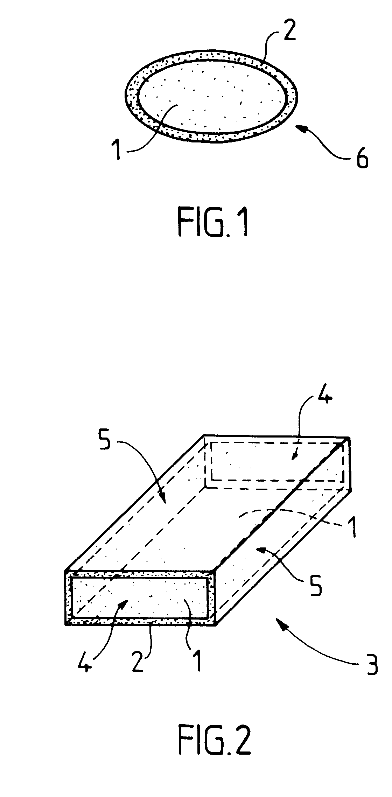

Selon un premier mode de réalisation, la couche d'enrobage 2 recouvre entièrement le noyau 1. L'assemblage de matières thermoplastiques pour épilation selon l'invention peut prendre toute forme appropriée, telle que par exemple grain, pastille ou pain. La figure 1 montre une vue en coupe d'un exemple de réalisation d'un assemblage de matières thermoplastiques pour épilation selon l'invention sous forme d'une pastille 6.

L'enrobage du noyau 1 pour former la couche 2 est obtenue par trempage ou par toute technique en milieu solide, liquide ou gazeux connue dans les domaines alimentaires ou pharmaceutiques.

Selon un deuxième mode de réalisation, la couche d'enrobage 2 est discontinue et laisse apparaître à la surface de l'assemblage de matières thermoplastiques le noyau 1. Selon ce mode de réalisation l'assemblage de matières thermoplastiques se présente de manière préférée sous la forme d'un pain. Avantageusement le pain 3 comporte deux faces d'extrémité 4 et au moins une face latérale 5.

Tel que représenté à la figure 2, le pain 3 présente une forme sensiblement parallélépipédique et comporte deux faces d'extrémité 4 et quatre faces latérales 5.

De manière avantageuse la couche d'enrobage 2 recouvre entièrement les faces latérales 5.

De manière préférée les deux faces d'extrémité 4 laissent déboucher le noyau 1. Avantageusement, tel que représenté à la figure 2 le noyau 1 débouchant des faces d'extrémité 4 est entouré par la couche d'enrobage 2.

Le pain 3 représenté à la figure 2 peut être obtenu par découpe d'un pain de longueur plus importante déjà recouvert de la couche d'enrobage 2.

A titre de variantes, non représentées aux figures, le pain 3 peut comporter deux faces d'extrémité 4 circulaires et une seule face latérale 5, ou encore deux faces d'extrémité 4 hexagonales et six faces latérales 5. D'autres formes sont bien entendu possibles.

A titre de variante complémentaire, non représentée aux figures, le noyau peut déboucher d'une seule des faces d'extrémité 4, l'autre face 4 étant entièrement recouverte par la couche d'enrobage 2.

Différentes matières peuvent être utilisées pour réaliser l'assemblage de matières thermoplastiques pour épilation selon l'invention.

Selon un premier exemple de réalisation, le noyau 1 est formé par une cire à épiler classique, d'origine végétale, présentant une plage de ramollissement de 70 à 80°C et la couche d'enrobage 2 est formée par une cire végétale telle que la cire de Carnauba, laquelle présente une plage de ramollissement de 82 à 84°C.

Selon un deuxième exemple de réalisation, le noyau 1 est formé par une cire à base sucre présentant une plage de ramollissement de 50 à 60°C, et la couche d'enrobage 2 est formée par une gomme végétale telle que la colophane, laquelle présente une plage de ramollissement de 70 à 80°C.

Selon un troisième exemple de réalisation, le noyau 1 est formé par une cire à base sucre présentant une plage de ramollissement de 50 à 60°C, et la couche d'enrobage 2 est formée par de la cire d'abeille, laquelle présente une plage de ramollissement de 60 à 65°C.

Selon un quatrième exemple de réalisation, le noyau 1 est formé par une cire à base sucre présentant une plage de ramollissement de 50 à 60°C, et la couche d'enrobage 2 est formée par de la cire de Candellila, laquelle présente une plage de ramollissement de 63 à 66°C.

A titre de variante d'autres matières thermoplastiques présentant une résistance mécanique suffisante pour former une couche autoporteuse peuvent être utilisées pour le noyau 1.

A titre de variante la couche d'enrobage 2 peut être formée par des mélanges de cires végétales ou minérales, de gommes végétales telles que la colophane, de polysaccharides, de protéines et/ou de composants de synthèse équivalents.

Des additifs peuvent être intégrés à la couche d'enrobage 2 pour améliorer les propriétés fonctionnelles : des plastifiants tels que polyols ou des lipides pour limiter les déformations, des émulsifiants tels que les mono et di-glycérides, et/ou des lécithines pour assurer la répartition des lipides et l'hydrophobicité, des acides tels que l'acide acétique ou l'acide citrique et/ou des bases pour la solubilisation, l'homogénéité et la résistance mécanique, des substances tensioactives pour l'adhésion et/ou la solubilisation.

## Revendications

1. Assemblage de matières thermoplastiques pour épilation, caractérisé en ce qu'il comporte un noyau (1) composé de cire à épiler et une couche d'enrobage (2) recouvrant au moins partiellement ledit noyau, ladite couche étant formée par une matière dont le point de ramollissement est plus élevé que celui de la cire dudit noyau.

2. Assemblage de matières thermoplastiques pour épilation selon la revendication 1, caractérisé en ce qu'il se présente sous la forme d'un pain (3).

3. Assemblage de matières thermoplastiques pour épilation selon la revendication 2, caractérisé en ce que le pain (3) comporte deux faces d'extrémité (4) et au moins une face latérale (5) .

4. Assemblage de matières thermoplastiques pour épilation selon la revendication 3, caractérisé en ce que le pain (3) est de forme sensiblement parallélépipédique et comporte deux faces d'extrémité (4) et 4 faces latérales (5).

5. Assemblage de matières thermoplastiques pour épilation selon l'une des revendications 3 ou 4, caractérisé en ce que la couche d'enrobage (2) recouvre entièrement la ou les faces latérales (5).

6. Assemblage de matières thermoplastiques pour épilation selon l'une des revendications 3 à 5, caractérisé en ce que l'une au moins des faces d'extrémité (4) laisse déboucher le noyau (1).

7. Assemblage de matières thermoplastiques pour épilation selon la revendication 6, caractérisé en ce que le noyau (1) débouchant de l'une des faces d'extrémité (4) est entouré par la couche d'enrobage (2).

8. Assemblage de matières thermoplastiques pour épilation selon la revendication 1, caractérisé en ce qu'il se présente sous la forme de grain ou de pastille.

9. Assemblage de matières thermoplastiques pour épilation selon l'une des revendications 1, 2 ou 8, caractérisé en ce que la couche d'enrobage (2) recouvre entièrement le noyau (1).

10. Assemblage de matières thermoplastiques pour épilation selon l'une des revendications 1 à 9, caractérisé en ce que l'épaisseur de la couche d'enrobage (2) disposée sur le noyau (1) est sensiblement constante.

11. Assemblage de matières thermoplastiques pour épilation selon l'une des revendications 1 à 10, caractérisé en ce que la couche d'enrobage (2) est à base de cire de Carnauba.

12. Assemblage de matières thermoplastiques pour épilation selon l'une des revendications 1 à 10, caractérisé en ce que la couche d'enrobage (2) est à base de colophane.

13. Assemblage de matières thermoplastiques pour épilation selon l'une des revendications 1 à 10, caractérisé en ce que la couche d'enrobage (2) est à base de cire d'abeille.

14. Assemblage de matières thermoplastiques pour épilation selon l'une des revendications 1 à 10, caractérisé en ce que la couche d'enrobage (2) est à base de cire de Candellila.

15. Assemblage de matières thermoplastiques pour épilation selon l'une des revendications 1 à 10, caractérisé en ce que le noyau (1) est formé par une cire à base sucre.
